# EUROPEAN PATENT APPLICATION

(11) **EP 4 574 094 A1**
(43) Date of publication of application: **25.06.2025**
(21) Application number: 23219168.4
(22) Date of filing: 21.12.2023
(51) Int. Cl.: A61F 2/24

(54) **STENT AND REPLACEMENT HEART VALVE PROSTHESIS WITH ONE CELL DESIGN**

(71) Applicant: Tricares GmbH, 85609 Aschheim (DE)
(72) Inventor: RAHMIG, Georg, 75175 Pforzheim (DE); STRAUBINGER, Helmut, 85609 Aschheim (DE)
(74) Representative: Hoefer & Partner Patentanwälte mbB

(57) **Abstract**

The present invention relates to a novel stent and a replacement heart valve prosthesis.

## Description

The present invention relates to a novel stent and a replacement heart valve prosthesis.

In the last decades minimally invasive techniques and catheter-based implantation techniques have advanced and are now feasible in many medical fields.

In a number of medical fields it is now possible to treat patients by catheter-based techniques allowing for the treatment of such patients who could otherwise not be adequately taken care of due to their physical condition and the risks connected with surgery. Such catheter-based techniques apply to delivery systems, e.g. a catheter and/or introducer sheath, for implanting the medical device to a desired target site via different access routes into a patient's body.

In particular, in recent years the treatment of heart valve diseases and deficiencies has become more and more successful. Examples are trans-apical, trans-jugular and trans-femoral procedures for heart valve replacement therapies, e.g. aortic or mitral heart valve treatments.

In many cases a stent-based prosthesis with a tissue based replacement valve is used and implanted to replace the native heart valve using a catheter delivery system.

The replacement heart valve prosthesis has to be crimped and loaded onto the catheter. The correct positioning of a replacement heart valve prosthesis, durability and good compliance with the target site and the biology of the target site are important aspects of such a prosthesis.

Hence there exists a need for a replacement heart valve prosthesis which can be correctly positioned, exhibits good durability features and has as little impact on or interference with the target site as possible and which remains long term correctly positioned. In addition, a replacement heart valve prosthesis should exhibit good sealing characteristics to avoid e.g. paravalvular leakage.

In particular in soft endogenous tissue like the mitral or tricuspid heart valve, and particularly in the tricuspid context which implies various challenges for a replacement heart valve design due to its soft tissue and prosthesis pressure requirements, it is difficult to properly position a replacement heart valve prosthesis and provide sufficient fixation properties as well as to avoid unnecessary interference with the endogenous valve environment.

More particularly, it is a problem to durably position the replacement heart valve, e.g. in tricuspid or mitral replacement heart valve technology and more so in the tricuspid heart valve context, wherein the diameter of the valve is large and the annulus and atrial and ventricular tissue surrounding the heart valve is either sensitive or anatomically challenging for precise positioning and fixation.

Accordingly, it is one object of the current disclosure to provide a replacement heart valve prosthesis, and in particular a tricuspid replacement heart valve prosthesis, with sufficient positioning and fixation features or/and exhibiting reduced interference characteristics at the target site, or at least to provide a prosthesis wherein the disadvantages of the prior art are essentially avoided or which show reduced disadvantages vis-à-vis the prior art.

Accordingly, it is another object of the current disclosure to provide a replacement heart valve prosthesis useful for replacement of the tricuspid heart valve functionality and to provide *inter alia* for an advantageously adapted tricuspid replacement heart valve as far as radial force is concerned having implications for fixation and functionality of the replacement heart valve and to advantageously influence compliance in the tricuspid context.

### Brief Summary of the Disclosure

In one aspect the disclosure relates to a stent or replacement heart valve prosthesis exhibiting advantageous positioning characteristics, or/and good durability characteristics or/and reduced interference characteristics, preferably in the tricuspid context.

In one aspect the disclosure relates to a stent structure useful for a replacement heart valve having a single cell structure from proximal (atrial end) to the distal (ventricular end), wherein the circumference of the stent structure is having a number of single cell structures, preferably 8 to 120, more preferably 10 - 20, most preferably 12 or 18, wherein the single cell structure is composed of two atrial intermediate struts (006), two ventricular intermediate struts (008), two longitudinal struts (007).

In one aspect the disclosure relates to a stent structure useful for a replacement heart valve as described above, wherein the dimensions of the struts is predefined, wherein a strut width apex (011), a strut width waist (012) and a strut radius (013) is predefined, preferably wherein the strut width apex (011) is from 0.30 mm to 0.36 mm wide, a strut width waist (012) is from 0.27 mm to 0.33 mm wide and a strut radius (013) is from 1150 mm to 2150 mm.

In one aspect the disclosure relates to a stent structure useful for a replacement heart valve and in particular in the tricuspid context having a single cell structure from proximal (atrial end) to the distal (ventricular end), wherein the circumference of the stent structure is having a number of single cell structures, preferably 8 to 120, more preferably 10 - 20, most preferably 12 or 18, wherein the single cell (005) structure is composed of two atrial intermediate struts, two ventricular intermediate struts, two longitudinal struts, wherein the dimensions of the struts is predefined, wherein a strut width apex, a strut width waist and a strut radius is predefined, preferably wherein the strut width apex is from 0.30 mm to 0.36 mm wide, a strut width waist is from 0.27 mm to 0.33 mm wide and a strut radius is from 1150 mm to 2150 mm and wherein the predefined intermediate struts are useful for defining essentially the radial force during expansion of the stent structure.

In another aspect the disclosure relates to a stent or replacement heart valve prosthesis characterized by an inner stent combined with an outer stent wherein fixation means are connected to or form part of the inner stent.

In another aspect the disclosure relates to a replacement heart valve prosthesis comprising an inner stent and an outer stent wherein fixation means are connected to or form part of the inner stent, and at least one sealing means connected to the inner and/or outer stent and a valve connected to the inner stent.

In another aspect the disclosure relates to method for implantation or positioning a replacement heart valve prosthesis in a native tricuspid or mitral heart valve wherein the prosthesis is delivered by a catheter-based delivery system to the target site.

In another aspect the disclosure relates to a method of replacement of a malfunctioning endogenous heart valve or the implantation of a replacement heart valve prosthesis in a person experiencing impaired heart valve function.

### Brief Description of the Drawings

Various embodiments of the disclosure are exemplified by the Figures wherein:

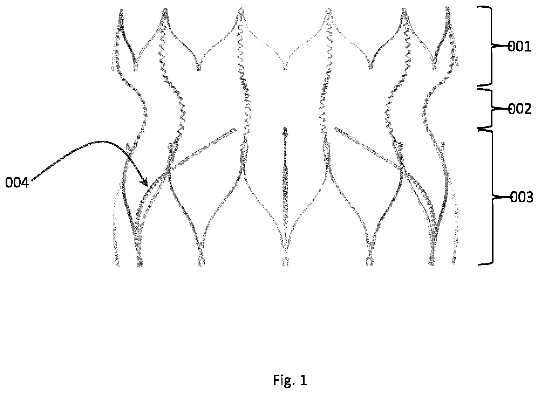

Fig. 1 illustrates an example of a stent (outer stent) according to the disclosure in a side view.

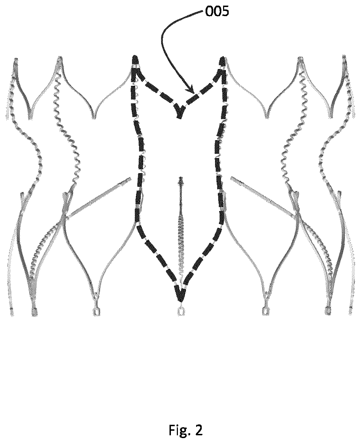

Fig. 2 illustrates an example of a stent (outer stent) according to the disclosure in a side view wherein one call is illustrated showing the single cell (005) structure composed of two atrial intermediate struts (006), two ventricular intermediate struts (008), two longitudinal struts (007).

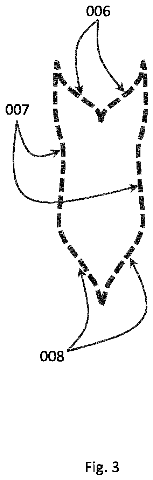

Fig. 3 depicts an isolated single cell (005) of a stent (outer stent) according to the disclosure showing the single cell (005) structure composed of two atrial intermediate struts (006), two ventricular intermediate struts (008), two longitudinal struts (007).

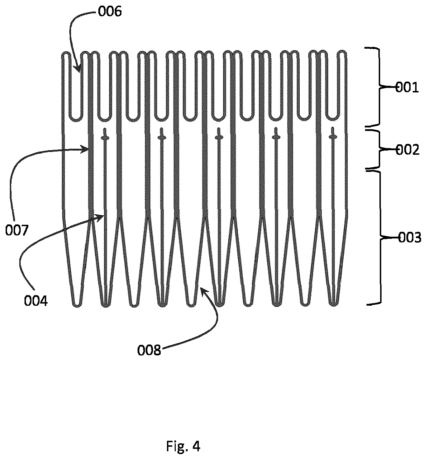

Fig. 4 depicts a laser cut stent in a side view of a stent according to the disclosure wherein the elements atrial section (001), middle section (V or U-groove) (002), ventricular section (003), connecting arm (strut) (004), single cell (005), atrial intermediate struts (006), longitudinal struts (007) and ventricular intermediate struts (008) is depicted as one example.

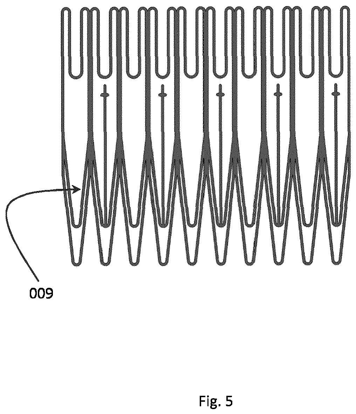

Fig. 5 depicts a variation of Fig. 4 including in addition ventricular reinforcement struts (009).

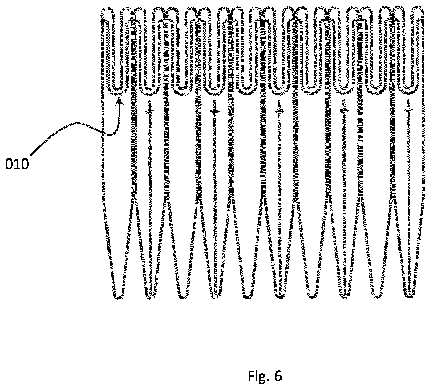

Fig. 6 depicts a variation of Fig. 4 including in addition atrial reinforcement struts (010).
Fig. 7 is a blow up of a detail of the embodiment as depicted in Fig. 6 showing details of elements (011, 012, 013).

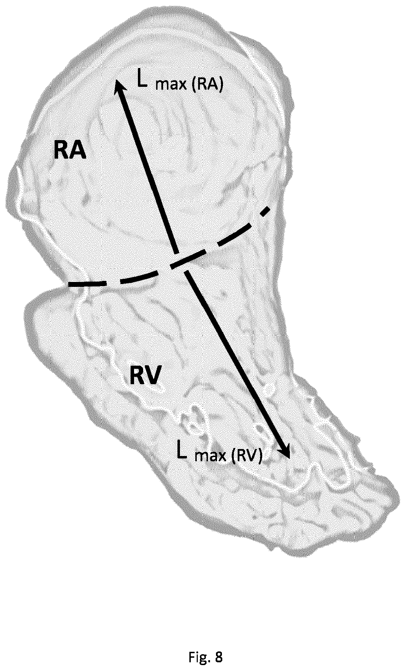

Fig. 8 shows a detail of the heart and the right heart side.

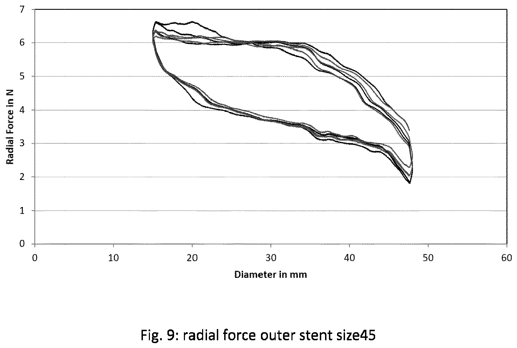

Fig. 9 is a graphic representation of radial force outer stent size 45.

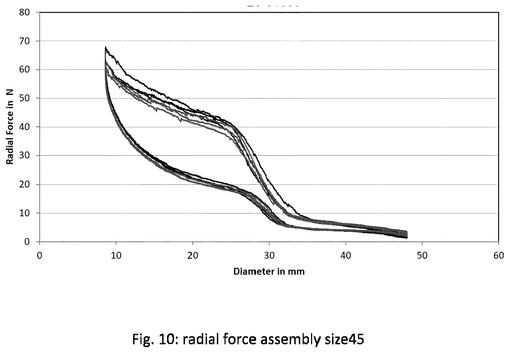

Fig. 10 is a graphic representation of radial force stent assembly (inner and outer stent) size 45.

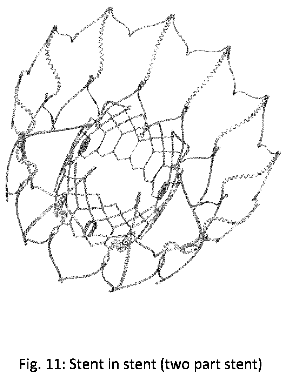

Fig. 11 illustrates an example of a stent assembly (inner stent and outer stent) according to the disclosure in a trimetric view.

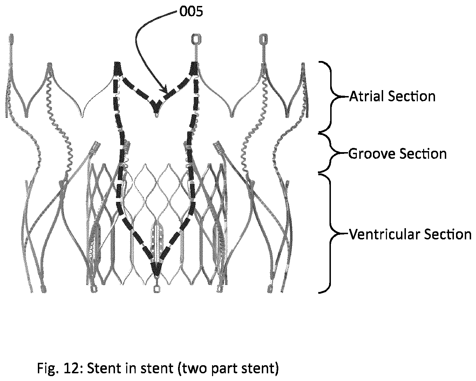

Fig. 12 illustrates an example of a stent assembly (inner stent and outer stent) according to the disclosure in a side view wherein one call is illustrated showing the single cell (005).

### Detailed Description

In the following certain terms of the disclosure will be defined. Otherwise technical terms in the context of the disclosure shall be understood as by the applicable skilled person.

The term "prosthesis" or "medical device" or "implant" in the sense of the disclosure is to be understood as any medical device that can be delivered in a minimally invasive fashion or by way of a catheter based procedure. The terms can be used interchangeably. A prosthesis in the sense of the disclosure can be e.g. a stent or stent-based prosthesis or stent-based replacement heart valve prosthesis like a mitral replacement heart valve or a tricuspid replacement heart valve.

The term "catheter" or "delivery device" in the sense of the disclosure is to be understood as the device used to deploy a prosthesis in a patient at a determined site, e.g. to replace a heart valve like a native aortic heart valve, mitral heart valve or tricuspid heart valve.

A "laser cut stent" in the sense of the disclosure is a stent which is laser cut from e.g. a nitinol tube.

A "stent area" or "stent areas" in the sense of the disclosure is a defined area of the outer stent, mesh stent or the replacement heart valve prosthesis and in particular it is a longitudinal section or an outer section defined as proximal, middle or distal area or atrial, annular or ventricular.

A "proximal area", "middle area", "distal area" in the sense of the disclosure denotes areas of the stent or prosthesis in relation to the operator performing implantation by use of a catheter wherein proximal is close to the operator and distal is away from the operator. "Middle area" denotes in a stent or prosthesis in the sense of the disclosure is the area between the distal and proximal area. The "proximal area" can also be denoted inflow end or inflow area and the "distal area" can also be denoted outflow end or outflow area with regards to the natural blood flow in situ, i.e. in vivo, in an individual (person or patient); proximal can also be denoted atrial, middle can be denoted annular and distal can be denoted ventricular.

An "annulus area" or "annular area" in the sense of the disclosure is either the respective area of an endogenous heart valve or it defines the respective area in the replacement heart valve or stent which is to be positioned at the implantation site and it meant to align with the endogenous annulus.

A "sub-annular area" in the sense of the disclosure is the area of the prosthesis which is in distal direction (or in inflow direction or in ventricular direction) of the annulus of the endogenous heart valve. The prosthesis may cover the "sub-annular area" with the U or V grove area and the distal area.

A "groove" in the sense of the disclosure describes an area of the stent or of the prosthesis exhibiting a smaller diameter than other areas and wherein distally and proximally of said groove other areas of the stent or prosthesis having a larger diameter are in neighborhood to said groove; said groove can have a V or U shape or combinations thereof or any other bended and useful geometries or it can be characterized by just a smaller diameter as compared to the atrial and ventricular stent areas.

A "target site" in the sense of the disclosure is the location or place where the replacement heart valve prosthesis is to be implanted and where a dysfunction or malfunction shall be treated, e.g. at the annulus of a tricuspid or mitral heart valve.

A "connection" of the stents in the sense of the disclosure is the way of fixation of two stents to each other by way of suturing, by way of a clipping or clicking mechanism or any other useful manner or connecting means to attach the stents or stent and fixation means to each other.

A "connecting means" in the sense of the disclosure is a mechanical or physical connection of two parts of a stent or laser cut stent. The connecting means can be by e.g. welding, gluing or any other known procedure or process or means. A connecting means can also be an attachment or clipping means which exhibits a special design and geometry for releasable or non-releasable connection.

"Fixation means" or "anchoring means" in the sense of the disclosure is a component connected with the inner stent and providing essentially for anchoring the prosthesis at the target site, optionally in cooperation with additional means. In a particular aspect a fixation means is composed of or comprises an anchoring loop, an anchoring arm, a connecting arch and an inner stent anchor, preferably a connecting means connects the inner stent anchor of the fixation means with the inner stent.

An "anchoring loop" in the sense of the disclosure is a part of a stent useful in fixation of a stent or prosthesis and which aids in avoiding movement of the stent or prosthesis at the target site. In general an anchoring loop in the sense of the disclosure is a means useful for an improved fixation of a stent or prosthesis wherein a loop is fixed to or connected with or forms part of or is an integral part of the inner stent. The "loop" or "loops" in the sense of the disclosure can have different shapes like round, square etc. and are located in a defined area in a defined pattern. A "loop" in the sense of the disclosure will exhibit a defined angle with regard to the inner stent surface and it may be designed that it may stretch out straight or flip over when the stent or prosthesis is retrieved into the catheter after an initial and possibly partial deployment.

An "angle structure" or "angle" in the sense of the disclosure is an angle between two accessory lines drawn at a certain area or stent layer in order to define a certain geometry of said stent part or arch or layer with regard to other stent structures like the inner stent.

A "radial force" in the sense of the disclosure is the force exhibited by a stent or prosthesis in radial outward direction, more particularly the force exhibited by the outer stent of the prosthesis which may be a laser cut stent, e.g. a nitinol stent. The radial force depends on the particular mesh or cut stent design and relates to the material density, e.g. the density of wires per square area in a mesh stent, or the number of cells and size of said cells circumferentially in a certain laser cut stent level or area, e.g. the proximal/atrial, middle/annular or distal/ventricular area. The radial force in a replacement heart valve prosthesis according to the disclosure will be chosen for the outer stent or the combination of the inner and outer stent and fixation means in a magnitude to provide for good contact with the surrounding tissue and to support the fixation functionality of the stent or prosthesis and optionally also as little as possible in order to avoid an interference with the endogenous environment and biology of the target site. Thus it will be chosen in a magnitude in order to avoid interference with the implantation site and endogenous tissue and function. Hence the stent and prosthesis according to the disclosure can be designed to achieve advantageous compliance with the requirements of the tricuspid valve environment. In the context of the disclosure the radial force is measured and defined as the radial force during expansion as measured according to the disclosure herein. The radial force may be supported for its fixation function by other means, e.g. loops for fixation.

The "target area" in the sense of the disclosure is the three-dimensional space surrounding or being within the native organ like a native heart valve which can be e.g. a tricuspid or mitral heart valve.

An "atraumatic design" of the loops in the sense of the disclosure is wherein the loops or other means or parts of a stent or a prosthesis are designed to avoid any or essentially any damage of the surrounding tissue or tissue in contact with said parts or at least parts manufactured in a manner to minimize damaging or/and injuring the tissue which they contact.

"Compliance" of the stent or replacement heart valve prosthesis, e.g. comprising an inner laser cut stent within an outer mesh stent, or a laser cut inner stent within a laser cut outer stent, in the sense of the disclosure relates to a positive interference with the target tissue. "Compliance" relates to a design which exhibits good geometry adaptation of the stent or prosthesis to the implantation site and wherein the stent or prosthesis exhibits advantageous fixation characteristics, good functionality as concerns valve function and at the same a minimal interference with the endogenous heart structures and heart function.

A "stent" in the sense of the disclosure is a stent of any suitable material e.g. nitinol laser cut stent which may be a single stent or a stent composed of an outer and an inner stent wherein the stents are connected by suitable means; the connection of the two stents may also be suitably comprise connecting arms and preferably connecting means which may preferably releasable connect the inner and outer stent. The sent is composed in its circumference of single cells wherein the number of single cells may vary depending on the shape, seize and other requirements of the stent, the number may be e.g. between 8 to 50, 10 to 20, 12 to 18, or any other suitable one cell number. The stent struts can be laser cut straight struts or e.g. longitudinal struts (007) can be designed as s-struts or/and wherein the longitudinal struts (007) comprise outwardly directed means, preferably hooks, preferably round shaped. Another possible design feature can be wherein the atrial intermediate struts (006) are forming a convex V shape or/and the ventricular intermediate struts (008) are forming a concave V shape, or/and the atrial intermediate struts (006) are directed inwardly in the stent cell or/and the ventricular intermediate struts (008) are directed outwardly of the stent cell. It is also possible that the outer stent can contain as another design feature ventricularily or/and atrially one or more eyelets.

"Single cell" or "single cell design" or "one cell" or "one cell design" in the sense of the disclosure is composed of two atrial intermediate struts, two ventricular intermediate struts, two longitudinal struts, wherein the dimensions of the struts is predefined.

"Strut width apex" in the sense of the disclosure is a part of a single cell of the stent and defines the width of a certain position of a strut, preferably at an end of a strut useful for defining the radial expansion force.

"Strut width waist" in the sense of the disclosure is a part of a single cell of the stent and defines the width of a certain position of a strut, preferably at the most narrow position of a strut useful for defining the radial expansion force.

"Strut radius" in the sense of the disclosure is a part of a single cell of the stent defines a predefined radius over the length of a strut between two defined points useful for defining the radial expansion force.

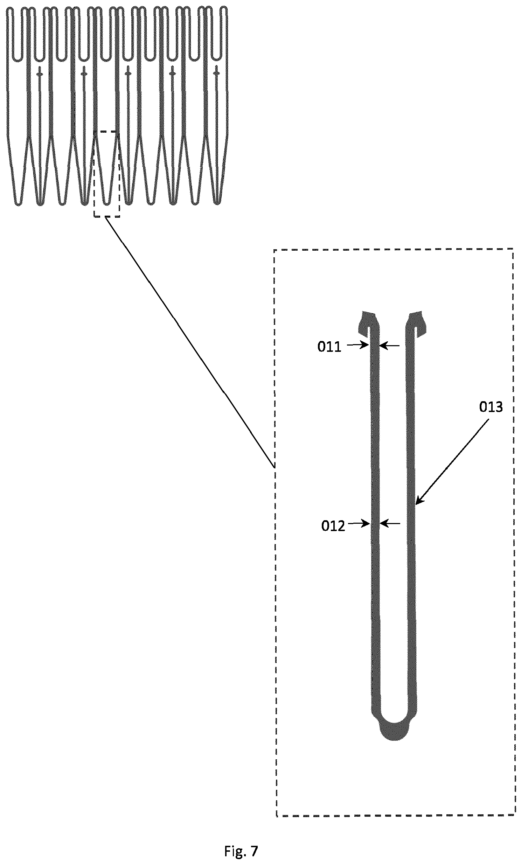

The stent elements "Strut width apex", "Strut width waist" and "Strut radius" are e.g. depicted in an example of the disclosure in Fig. 7. These three parameters can be used to design and define the radial expansion force.

"Atrial or/and ventricular reinforcement struts" (010, 009) in the sense of the disclosure is a means which is useful for influencing the stability of a certain strut, preferably it increases the stability or/and reduces the flexibility of a strut or a region in a stent or/and a single cell as described herein. A reinforcement strut can be a strut which is located at the atrial or/and ventricular area of a stent or/and a single cell of a stent, e.g. an additional strut can be positioned parallel to an atrial or/and ventricular strut of a single cell. It is possible that there is one, two or three additional struts positioned.

"Radial force during expansion" in the sense of the disclosure is the radial force which can be measured on the outer stent from a crimped stage to an expanded stage measured with the method as disclosed herein (according ASTM standard F3067-14). Preferably the predefined intermediate struts are useful for defining essentially the radial force during expansion of the stent structure. It may be further influenced by various design features and stent composition, i.e. characterized by a single stent or the stent being composed of an outer and inner stent which will have an influence on the radial force during expansion.

In one aspect of the disclosure, the problem underlying the application is solved by claims 1, 13 or 15.

In one aspect of the disclosure, the problem underlying the application is solved by a stent being composed of single cells wherein the design of one single cells consists of two atrial intermediate struts, two ventricular intermediate struts, two longitudinal struts, wherein the dimensions of the struts is predefined and wherein a strut width apex (011), a strut width waist (012) and a strut radius (013) is predefined, and wherein the predefined struts are useful for defining essentially the radial force during expansion of the stent structure.

In one aspect the disclosure relates to a stent structure useful for a replacement heart valve having a single cell (005) structure from proximal (atrial end) to the distal (ventricular end), wherein the circumference of the stent structure is having a number of single cell (005) structures, preferably 8 to 120, more preferably 10 - 20, most preferably 12 or 18, wherein the single cell (005) structure is composed of two atrial intermediate struts (006), two ventricular intermediate struts (008), two longitudinal struts (007), wherein the dimensions of the struts is predefined, wherein a strut width apex (011), a strut width waist (012) and a strut radius (013) is predefined, preferably wherein the strut width apex (011) is from 0.30 mm to 0.36 mm wide, a strut width waist (012) is from 0.27 mm to 0.33 mm wide and a strut radius (013) is from 1150 mm to 2150 mm and wherein the predefined intermediate struts are useful for defining essentially the radial force during expansion of the stent structure.

In one aspect the disclosure relates to a replacement heart valve prosthesis comprising or consisting of a stent structure as described herein and a sealing means.

In one aspect the disclosure relates to a method for treating a tricuspid heart valve disorder or deficiency by minimally invasive implantation of a replacement heart valve prosthesis as described herein or/and treating a person experiencing impaired heart valve function using a replacement heart valve prosthesis as described herein.

Hence in one aspect the invention provides for advantageous anchoring characteristics and/or an advantageous compliance with the heart and the structures and other valves of the heart wherein the stent or/and prosthesis is implanted and in particular with the tricuspid functionality.

In another advantageous aspect the stent and prosthesis according to the disclosure provides for a sealing by way of the outer stent and the related sealing materials attached thereto which serves the primary purpose of sealing and avoiding paravalvular leakage. The sealing on the outer stent is preferably on the inside of the outer stent. In another embodiment said sealing is on the outside of the outer stent. The special design of the prosthesis advantageously decouples the outer stent from the basic anchoring functionality provided by the anchoring means and which is essentially decoupled from the outer stent. Thus the outer stent serves essentially for sealing and the sealing functionality is decoupled from the anchoring functionality in turn may result in an improved sealing characteristics by a better alignment of the outer sealing stent. Moreover, the inner stent carrying a replacement heart valve attached to the inner stent and comprising preferably additional sealing is connected to the outer stent and by way of its special strut connecting means is decoupled from the heart beat impact on the outer stent, and hence the functionality of the inner stent and its valve is improved.

The advantageous sealing characteristics of the outer stent and advantageous alignment with the surrounding tissue of the target site may be further achieved not only by the decoupling but also by the use of a one cell design of the laser cut outer stent which advantageously has a reduced stiffness and/or increased flexibility as compared to laser cut inner stent.

Preferred embodiments according to the disclosure are described in the dependent claims.

Preferred embodiments are described in the dependent claims.

In particular, preferred embodiments relate to a stent structure as described herein further comprising atrial or/and ventricular reinforcement struts (010, 009). The stent structure as described herein can preferably be characterized by three sections, an atrial section (001), a middle section (002) and a ventricular section (003), wherein the middle section (002) is a V or U groove. The stent structure as described herein can preferably be characterized, wherein the radial force during expansion of the stent structure measured in a target diameter of 35 mm to 55 mm diameter is from 2 to 6 N, preferably from 2.5 to 4 N measured according ASTM standard F3067-14. The sent structure as described herein can preferably be characterized, wherein the radial force during expansion of the stent structure measured in a target diameter of 35 to 45 mm diameter is from 2 to 6 N, preferably from 2.5 to 4 N, or in a target diameter of 45 to 55 mm diameter is from 2 to 6 N, preferably from 2.5 to 4 N measured according ASTM standard F3067-14. The stent structure as described herein can preferably be characterized, further comprising an inner stent wherein a replacement valve is attached to the inner stent and wherein the inner stent is connected to the outer stent by way of connecting arms (004) and preferably a connecting means, preferably wherein the connecting means are positioned proximally of the inner stent, preferably in the annulus or atrium. The stent structure as described herein can preferably be characterized, wherein the connecting arms (004), preferably 4, 5, 6, 7, 8, 9, 10, 11, or 12, connect the inner stent at its atrial area with the outer stent at its ventricular area, preferably at the outer most atrial and/or at the outer most ventricular ends, preferably wherein the connecting arms (004) comprise S strut regions or wherein the connecting arms (004) are S struts. The stent structure as described herein can preferably be characterized, wherein the radial force during expansion of the stent structure measured on the outer stent in a target area of 35 mm to 55 mm diameter is from 2 to 6 N, preferably from 2.5 to 4 N measured according ASTM standard F3067-14. The stent structure as described herein can preferably be characterized, wherein the radial force during expansion of the stent structure measured on the outer stent in a target area of 35 to 45 mm diameter is from 2 to 6 N, preferably from 2.5 to 4 N, or in a target area of 45 to 55 mm diameter is from 2 to 6 N, preferably from 2.5 to 4 N measured according ASTM standard F3067-14. The stent structure as described herein can preferably be characterized, wherein one or more of the longitudinal struts (007) are s-struts or/and wherein the longitudinal struts (007) comprise outwardly directed means, preferably hooks, preferably round shaped. The stent structure as described herein can preferably be characterized, wherein the atrial intermediate struts (006) are forming a convex V shape or/and the ventricular intermediate struts (008) are forming a concave V shape, or/and the atrial intermediate struts (006) are directed inwardly in the stent cell or/and the ventricular intermediate struts (008) are directed outwardly of the stent cell. The stent structure as described herein can preferably be characterized in that the outer stent contains ventricularly or/and atrially one or more eyelets.

The replacement heart valve as described herein can preferably be characterized as a prosthesis comprising or consisting of a stent structure as described herein and a sealing means useful for treating a tricuspid heart valve disorder or deficiency.

One aspect of an embodiment according to the disclosure is a stent comprising an inner stent and an outer stent wherein the two stents are connected with at least one or more, preferably 4 to 20, more preferably with 10 to 12 connecting struts and preferably connecting means combining the connecting struts originating from the inner and outer stent, respectively.

A more specific description of aspects of the disclosure is a replacement heart valve prosthesis, a method of implanting such a prosthesis and a method of positioning such a prosthesis.

In particular in another aspect the problem underlying the application is solved by a replacement heart valve prosthesis comprising an inner stent and an outer stent as describe above and at least one sealing means in the inner and/or outer stent and a valve connected to the inner stent, wherein the valve has optionally 2 or 3 leaflets.

In another aspect the problem underlying the application is solved by a method for implanting a replacement heart valve prosthesis as describe above using a catheter based delivery system.

In another aspect the problem underlying the application is solved by a method for positioning a replacement heart valve prosthesis in a native tricuspid or mitral heart valve wherein the prosthesis is delivered by a_catheter based delivery system to the target site (e.g. the native mitral or tricuspid annulus) wherein the target site is the annulus, the prosthesis get released from the delivery system into the right or left heart chamber. Preferred is a tricuspid prosthesis.

In a stent according to the disclosure the inner stent exhibits a relatively high stiffness and the outer stent exhibits a relatively low stiffness and has a relatively high flexibility in order to adapt and align with the target endogenous tissue. Thus it has been shown to be advantageous if the outer stent has a higher flexibility compared to the inner stent. Thus the inner stent has a higher stiffness as compared to the outer stent. In the outer stent the flexibility and stiffness can be designed due to its one cell design and a choice of the two atrial intermediate struts (006), two ventricular intermediate struts (008), two longitudinal struts (007), wherein the dimensions of the struts is predefined, wherein preferably a strut width apex (011), a strut width waist (012) and a strut radius (013) is predefined, and wherein the predefinition of the dimensions of the aforementioned structures can define the flexibility of the outer stent.

In particular the stent can be designed with regards to the one cell design in a way so that one achieves a radial force during expansion at 35 to 45 mm expansion, preferably at 45 mm expansion of the stent a can be designed in a way so that one achieves a radial of between 6 and 2 N, preferably of between 4 and 2.5 N wherein the radial force is measured according to ASTM standard F3067-14. Or one can design the particular struts of the one cell design in a way so as to achieve a radial force during expansion at 45 to 55 mm expansion, preferably at 55 mm expansion of the stent so that one achieves a radial force during expansion of between 6 and 2 N, preferably of between 4 and 2.5 N wherein the radial force is measured according to ASTM standard F3067-14. Preferably the radial force during expansion is 6 - 7 N at 15 mm expansion, or/and 2.8 N at 45 mm expansion.

In particular the stent can be designed with regards to the one cell design in a way so that one achieves a radial force during expansion at 35 to 45 mm expansion, preferably at 45 mm expansion of the stent a can be designed in a way so that one achieves a radial of between 6 and 2 N, preferably of between 4 and 2.5 N wherein the radial force is measured according to ASTM standard F3067-14.

Hence, the one cell design in the stent or outer stent and in particular the two atrial intermediate struts (006), two ventricular intermediate struts (008), two longitudinal struts (007), preferably the strut width apex (011), strut width waist (012) and strut radius (013) is useful to define the radial force of the stent structure and prosthesis. In particular the struts of the one cell of the stent and the particular design, and dimensions with regard to preferably the strut width apex (011), strut width waist (012) and strut radius (013) is useful to define the radial force during expansion of the stent structure and prosthesis. The preferred material is nitinol in such a stent structure.

The radial force during expansion can further be influenced by inclusion of atrial or/and ventricular reinforcement struts (010, 009

The radial force during expansion can be varied by way of inclusion or/and variation of the number of strut width apex (011), strut width waist (012) and strut radius (013) is varied..

By way of addition of reinforcement struts in particular at a specific area of the outer stent, e.g. in the proximal or/and atrial or/and groove area, the radial force during expansion can be designed and set to a defined value over the length of the stent and thus prosthesis.

In case several reinforcement struts are used over the length of the stent or prosthesis the radial force during expansion can be changed in a step wise manner by way of changing the parameter strut width apex (011), strut width waist (012) and strut radius (013). As well or in an alternative the length of the stent or prosthesis will be a means to change the radial force during expansion.

Moreover in a preferred embodiment in the stent structure according to the disclosure the atrial end of the stent is designed in an undulating or/and meander manner and/or having the two longitudinal struts (007) designed as s-struts and wherein the two atrial intermediate struts (006) are concave in relation to the two longitudinal struts (007) and/or the two ventricular intermediate struts (008) are convex in relation to the two longitudinal struts (007). Moreover, in a preferred embodiment atrial or/and ventricular reinforcement struts (010, 009) can be included in order to increase the radial force of the stent. In particular useful is a variation in the distal part of the stent e.g. loops in the distal direction. In the same manner can additional struts in the proximal stent area increase the radial force.

In particular the strut width apex (011), strut width waist (012) and strut radius (013) are useful to define and modify the radial force during expansion of the stent structure and prosthesis. The strut radius (013) is chosen preferably concave.

By modifying the strut width apex (011), e.g. by increasing the strut width apex (011) one can achieve an increase of the radial expansion force.

By modifying the strut width apex (011), e.g. by decreasing the strut width apex (011) one can achieve a decrease of the radial expansion force.

By modifying the strut width waist (012), e.g. by increasing the strut width waist (012) one can achieve an increase of the radial expansion force.

By modifying the strut width waist (012), e.g. by decreasing the strut width waist (012) one can achieve a decrease of the radial expansion force.

By modifying the strut radius (013), e.g. by increasing the strut radius (013) one can achieve an increase of the strut width waist (012) and herewith an increase of the radial expansion force.

By modifying the strut radius (013), e.g. by decreasing the strut radius (013) one can achieve a decrease of the strut width waist (012) and herewith an increase of the radial expansion force.

In a preferred embodiment, the structures contributing essentially to the radial force during expansion and thus being important for fixation of the stent or/and prosthesis is the design of the two atrial intermediate struts (006) and the two ventricular intermediate struts (008).

In one preferred embodiment the stent is positioned in the annulus with the groove and the stent is useful for anchorage in the annulus by the radial force during expansion as described above. Additional hooks or loops may be useful for increased anchoring.

In one embodiment according to the disclosure the inner stent is a laser cut stent and the outer stent is a laser cut stent or a braided stent, and wherein the inner stent and/or outer laser cut stent comprises 6 to 48 cells circumferentially and 1 cell longitudinally or the outer braided stent (1) comprises or is composed of 6 to 72 wire meshes circumferentially and 1 wire mesh longitudinally.

Moreover the skilled person will appreciate that he will chose appropriate materials for the stent structures which materials may be stainless steel, nitinol, plastics, composite materials or other known materials.

The stents according to the disclosure will be connected as it is useful in view of the entire design and functionality. A stent according to the disclosure in one embodiment is a stent wherein the inner stent and outer stent are connected to each other in the atrial area or at the atrial end.

In a stent according to the disclosure the connecting struts and/or the connecting means can be connected or positioned in the ventricular area or at the ventricular end or in the atrial area or at the atrial end of the inner stent.

It has been shown to be useful if the stent comprises means for an easier loading onto a catheter. In a stent according to the disclosure the outer stent can comprise at least two loading loops or/and a groove, e.g. a V or U groove (5).

Stent according to the disclosure can be defined in three sections, e.g. an atrial area, an annulus area and a ventricular area.

The dimensions of the stent will be adapted to each other and the dimensions of the target area and space. In a stent according to the disclosure in one embodiment can have dimensions wherein the atrial area has a diameter of 20 to 90 mm and a length of 2 to 30 mm, the annulus area has a diameter of 10 to 80 mm and a length of 2 to 20 mm and the ventricular area has a diameter of 20 to 90 mm and a length of 5 to 40 mm.

The number of connecting means can vary. In one embodiment of a stent according to the disclosure the number of connecting struts originating from each stent (inner and outer stent) and the number of connecting means is from 2 to 36.

In a preferred embodiment anchoring loops support the fixation at the target site. In one embodiment a stent or outer stent according to the disclosure comprises 1 to 54 anchoring loops.

The method as described above can be further characterized in that it relates to a method for implanting a replacement heart valve prosthesis using a catheter-based implantation and delivery system.

The catheter will be introduced as is appropriate with regards to the target native heart valve and the catheter system can be introduced trans-femoral, trans-atrial, trans-jugular, or trans-apical.

In another detail the disclosure relates to a method for positioning a replacement heart valve prosthesis in a native tricuspid or mitral heart valve wherein the prosthesis is delivered by a catheter based delivery system to the target site (e.g. the native mitral or tricuspid annulus), the prosthesis is released from the delivery system into the right or left heart chamber, wherein the annular or/and ventricular area includes anchoring loops and these are positioned essentially in the ventricle or essentially in the area of the annulus.

The prosthesis as disclosed herein can also be retrieved in case of sub-optimal positioning or other problems during the implantation procedure.

### Examples

The following examples serve to illustrate various embodiments of the disclosure. They are not meant to be interpreted as restrictive in any way.

### 1. Radial expansion force measuring method:

Radial Force tests were performed on a Radial Expansion Force Gauge (RX650) manufactured by Machine Solutions Inc. (MSI) with integrated high speed data acquisition system. The RX650 equipment uses an encoder for diameter accuracy, a linear actuator for activation and a precision roller bearing system providing a low friction testing environment. The measurement head of the MSI RX650 device uses 12 polished metallic elements from stainless steel like an iris orifice to crimp and/or expand a cylindrical stent component.

| Parameter / Features | Value / Specifications |
|---|---|
| Test Temperature | 37°C +/-2°C |
| Head length | 120 mm |
| Start diameter | 50 mm |
| Crimp diameter (outer stent) | 15 mm |
| Crimp diameter (assembly) | 15 mm |
| Diameter accuracy | 0.2% of full scale |
| Diameter resolution | 0.01 mm |
| Force repeatability | 1.0% of full scale |
| Force resolution | 0.06% of full scale |
| Measurement Speed | 0.1 mm/s |
| Number of Test Cycles | 3 |

### Reference Number List

- 001 -: atrial section
- 002 -: middle section (V or U-groove)
- 003 -: ventricular section
- 004 -: connecting arm (struts)
- 005 -: single cell
- 006 -: atrial intermediate struts
- 007 -: longitudinal struts
- 008 -: ventricular intermediate struts
- 009 -: ventricular reinforcement struts
- 010 -: atrial reinforcement struts
- 011 -: strut width apex
- 012 -: strut width waist
- 013 -: strut radius

## Claims

1. Stent structure useful for a replacement heart valve having a single cell (005) structure from proximal (atrial end) to the distal (ventricular end), wherein the circumference of the stent structure is having a number of single cell (005) structures , preferably 8 to 120, more preferably 10 - 20, most preferably 12 or 18, wherein the single cell (005) structure is composed of two atrial intermediate struts (006), two ventricular intermediate struts (008), two longitudinal struts (007), wherein the dimensions of the struts is predefined, wherein a strut width apex (011), a strut width waist (012) and a strut radius (013) is predefined, preferably wherein the strut width apex (011) is from 0.30 mm to 0.36 mm wide, a strut width waist (012) is from 0.27 mm to 0.33 mm wide and a strut radius (013) is from 1150 mm to 2150 mm and wherein the predefined intermediate struts are useful for defining essentially the radial force during expansion of the stent structure.

2. Stent structure according to claim 1 further comprising atrial or/and ventricular reinforcement struts (010, 009).

3. Stent structure according to claim 1 or 2 **characterized by** three sections, an atrial section (001), a middle section (002) and a ventricular section (003), wherein the middle section (002) is a V or U groove.

4. Stent structure according to any of claims 1 - 3, wherein the radial force during expansion of the stent structure measured in a target diameter of 35 mm to 55 mm diameter is from 2 to 6 N, preferably from 2.5 to 4 N measured according ASTM standard F3067-14.

5. Sent structure according to any of claims 1-4, wherein the radial force during expansion of the stent structure measured in a target diameter of 35 to 45 mm diameter is from 2 to 6 N, preferably from 2.5 to 4 N, or in a target diameter of 45 to 55 mm diameter is from 2 to 6 N, preferably from 2.5 to 4 N measured according ASTM standard F3067-14.

6. Stent structure according to any of claims 1 to 5, further comprising an inner stent wherein a replacement valve is attached to the inner stent and wherein the inner stent is connected to the outer stent by way of connecting arms (004) and preferably a connecting means, preferably wherein the connecting means are positioned proximally of the inner stent, preferably in the annulus or atrium.

7. Stent structure according to claim 6, wherein the connecting arms (004), preferably 4, 5, 6, 7, 8, 9, 10, 11, or 12, connect the inner stent at its atrial area with the outer stent at its ventricular area, preferably at the outer most atrial and/or at the outer most ventricular ends, preferably wherein the connecting arms (004) comprise S strut regions or wherein the connecting arms (004) are S struts.

8. Stent structure according to claim 6 or 7, wherein the radial force during expansion of the stent structure measured on the outer stent in a target area of 35 mm to 55 mm diameter is from 2 to 6 N, preferably from 2.5 to 4 N measured according ASTM standard F3067-14.

9. Stent structure according to claim 6 or 7, wherein the radial force during expansion of the stent structure measured on the outer stent in a target area of 35 to 45 mm diameter is from 2 to 6 N, preferably from 2.5 to 4 N, or in a target area of 45 to 55 mm diameter is from 2 to 6 N, preferably from 2.5 to 4 N measured according ASTM standard F3067-14.

10. Stent structure according to any of claims 1 to 9, wherein one or more of the longitudinal struts (007) are s-struts or/and wherein the longitudinal struts (007) comprise outwardly directed means, preferably hooks, preferably round shaped.

11. Stent structure according to any of claims 1 to 9, wherein the atrial intermediate struts (006) are forming a convex V shape or/and the ventricular intermediate struts (008) are forming a concave V shape, or/and the atrial intermediate struts (006) are directed inwardly in the stent cell or/and the ventricular intermediate struts (008) are directed outwardly of the stent cell.

12. Stent structure according to any one of the preceding claims **characterized in that** the outer stent contains ventricularily or/and atrially one or more eyelets.

13. Replacement heart valve prosthesis comprising or consisting of a stent structure according to any of the preceding claims and a sealing means.

14. Replacement heart valve prosthesis comprising or consisting of a stent structure according to any of the preceding claims and a sealing means useful for treating a tricuspid heart valve disorder or deficiency.

15. Method for treating a tricuspid heart valve disorder or deficiency by minimally invasive implantation of a replacement heart valve prosthesis according to claim 13 or/and treating a person experiencing impaired heart valve function using a replacement heart valve prosthesis according to claim 13.
